# EUROPEAN PATENT SPECIFICATION

(11) **EP 2 058 028 B1**
(45) Date of publication and mention of the grant of the patent: **03.04.2019**
(21) Application number: 07710937.9
(22) Date of filing: 13.02.2007
(51) Int. Cl.: A61N 7/00, A61B 5/055, A61B 8/13, A61N 7/02

(54) **A HIGH INTENSITY FOCUSED ULTRASOUND THERAPEUTIC SYSTEM GUIDED BY AN IMAGING DEVICE GUIDED**
VON EINEM BILDGEBUNGSGERÄT GEFÜHRTES HOCHINTENSIVES FOKUSSIERTES ULTRASCHALL-THERAPIESYSTEM
SYSTÈME THÉRAPEUTIQUE À ULTRASONS FOCALISÉS DE HAUTE INTENSITÉ GUIDÉ PAR UN DISPOSITIF D'IMAGERIE

(30) Priority: 24.08.2006 CN 200610111926
(43) Date of publication of application: 13.05.2009
(73) Proprietor: Chongqing Ronghai Medical Ultrasound Industry Ltd., Yubei District Chongqing 401121 (CN)
(72) Inventor: WANG, Hai, Chongqing 401121 (CN); WANG, Long, Chongqing 401121 (CN); ZHANG, Lian, Chongqing 401121 (CN); GU, Yue, Chongqing 401121 (CN)
(74) Representative: Cohausz & Florack
(86) International application number: PCT/CN2007/000512
(87) International publication number: WO 2008/025190

(56) References cited:
- EP-A1- 0 614 651
- WO-A1-2005/030330
- CN-A- 1 215 616
- CN-A- 1 814 320
- CN-Y- 2 370 887
- US-A- 6 128 522
- US-B1- 6 522 142
- US-B1- 6 522 142
- US-B1- 6 823 216
- CHUNG ANDREW H ET AL: "Thermal dosimetry of a focused ultrasound beam in vivo by magnetic resonance imaging", MEDICAL PHYSICS, AIP, MELVILLE, NY, US, vol. 26, no. 9, 1 September 1999 (1999-09-01), pages 2017-2026, XP012010913, ISSN: 0094-2405, DOI: 10.1118/1.598707
- YI Z., GUO Y., LU X.: 'MRI ULTRASOUND FOCAL KNIFE LIVER CANCER THERAPEUTIC EFFECT DISCUSSION' JOURNAL OF CHINESE PHYSICIAN vol. 8, no. 2, February 2006, pages 261 - 262, XP008104287

## Description

### FIELD OF THE INVENTION

The present invention pertains to the technical field of medical devices and relates to an ultrasound therapeutic system, particularly to a high intensity focused ultrasound therapeutic system guided by an imaging device.

### BACKGROUND OF THE INVENTION

In the field of ultrasound therapy, due to technology and awareness, people do not know much about the action mechanism of the focus in vivo and always expect to use multiple focuses to achieve a fast treatment when an ultrasound thermotherapy is applied for treatment. However, the therapeutic effects are not ideal when an ultrasound therapeutic apparatus applies such multiple focuses for treatment. Meanwhile, with the development of ultrasound technology, especially when the high intensity focused ultrasound is being used widely in therapy, a fine surgical treatment apparatus is in higher demand of people because only such apparatus can be used to treat or ablate some diseases (particularly the malignant tumors) completely.

As a therapeutic apparatus, at present, the high intensity focused ultrasound therapeutic apparatus has been widely applied in clinic at home and abroad. This therapeutic apparatus focalizes the extra-corporeal low-energy ultrasonic wave at the focus in vivo and consequently transient high temperature effects (60∼100 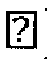 ), cavitation effects and mechanical effects are produced at the diseased tissue in the focal region and accordingly the cell and nuclear membranes are disrupted and the protein is coagulated. These effects are used to selectively cause coagulative necrosis of the diseased tissue and disable the proliferation, infiltration and metastasis of the diseased tissue; thereby the disease can be treated.

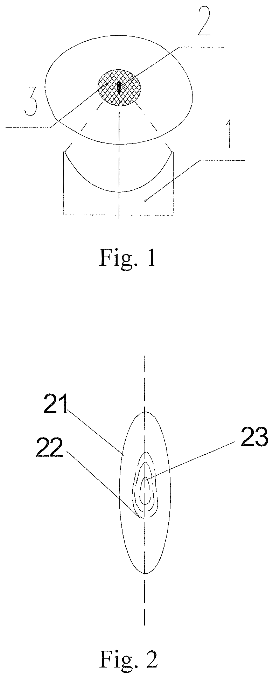

The principle of high intensity focused ultrasound therapy is shown in Fig. 1. The ultrasound transducer 1 emits ultrasonic waves and these ultrasonic waves go through the coupling medium and are focused inside the patient. The coagulative necrosis region formed is a biological focal region (i.e. BFR). BFR is an ellipsoid smaller than the diseased tissue 3. In order to treat the entire diseased tissue 3, BFR 2 needs to be localized within the diseased tissue 3 and then scans and treats the entire diseased tissue 3.

In order to monitor the process of high intensity focused ultrasound therapy, a B-mode scanner, a CT, an MRI or other equipments can be applied.

US patent No. 5,485,839 discloses a therapeutic system combining an MRI means with an ultrasonic therapeutic apparatus. This patent points out that the tissue such as tumor to be treated usually has a complicated three-dimensional shape, so it is very difficult to realize a complete treatment of the entire tumor and to evaluate the therapeutic effects simply by using the two-dimension ultrasonic tomographic images, and it presents a technical solution of utilizing the three-dimensional MRI tomographic images to image the object before and after treatment and utilizing two-dimensional ultrasound to monitor the treatment process in real time.

The patent as mentioned above utilized an MRI to establish three-dimensional images of the object to be treated before and after treatment and displayed the three-dimensional shape of the object created in the display. However, because the shape of tumor is irregular and usually is dendritic, some information at the ends will be lost when the three-dimensional images of the object to be treated are collected. Therefore, the three-dimensional images reconstructed in this way are not real and can not show the overall original form of the tumor. The treatment can not be carried out completely based on these distorted images. For malignant tumors, the remaining diseased tissue will continue to expand and accordingly the hazard to the life of the patient will occur again. So, the technical solutions of the patent mentioned above can not complete an accurate ultrasound therapy. Also, if the MRI apparatus in that patent is not reconstructed, the patient has to be moved in and out of the MRI bore repeatedly and thereby the treatment time is prolonged. If the patient is not moved, the structure of MRI has to be modified and thereby the treatment cost will be increased.

Further, document US 6 128 522 A discloses that magnetic resonance information acquired by a movable magnetic resonance instrument is used to monitor hyperthermia treatments such as tissue ablation. The instrument according to US 6 128 522 A may include both the magnetic resonance equipment and an energy applicator such as a high intensity focused ultrasound unit. From document "Thermal dosimetry of a focused ultrasound beam in vivo by magnetic resonance imaging", a MRI device with three-dimensional imaging function is known. Document EP 0 614 651 A1 discloses an ultrasonic wave medical treatment apparatus suitable for use under guidance of magnetic resonance imaging. Eventually, document US 6 522 142 B1 discloses systems and methods using magnetic resonance imaging for monitoring the temperature of a tissue mass being heated by energy converging in a focal zone that is generally elongate and symmetrical about a focal axis.

### SUMMARY OF THE INVENTION

Aiming at the above-mentioned disadvantages in the prior art, the technical solutions of the present invention are to provide a high intensity focused ultrasound therapeutic system guided by an imaging device, which can image the target accurately and can perform an accurate treatment for a relatively complicated operation. The invention is defined by the subject matter of claim 1.

The technical solutions for solving the problems in the present invention are as follows: this high intensity focused ultrasound therapeutic system guided by an imaging device comprises an ultrasound therapeutic applicator, which contains an ultrasound transducer, a therapeutic bed, on which a patient lies, and an imaging device used for imaging the diseased tissue. Wherein, said imaging device is a non-ultrasound imaging device with two-dimensional imaging function. The ultrasound transducer with the diameter of focal core ranging from 0.1mm to 5mm in the biological focal region is adopted. When the ultrasound transducer is manufactured, the diameter of the focal core in the biological focal region of the ultrasound transducer ranging from 0.1mm to 5mm can be determined by controlling the main parameters of the ultrasound transducer, for example, focal length, frequency, etc., then verifying through focal region test and experiments in vitro.

Preferably, said ultrasound transducer with the diameter of focal core ranging from 0.2mm to 2mm in the biological focal region located in the diseased tissue is adopted.

With the further and deeper studies on biological focal region, it has been found that in the entire biological focal region the temperature rise at the focal region center, that is, focal core, is the highest, and the energy degression at the outer edge of the focal center is sharp and with the characteristics of a "knife". For more information, please refer to *Investigation On Temperature Field Of Biological Focal Region Induced By High Intensity Focused Ultrasound* ("Chinese Journal of Biological Engineering", 2003, 22 (4) : 321-325, Faqi Li et al.). Fig. 2 is a longitudinal section diagram of a biological focal region. From this diagram, it can be seen that the biological focal region comprises three parts, i.e. focal edge 21, focal segment 22 and focal core 23. This study provides a theoretical support for a fine surgery and it makes people realize that the focal core 23 instead of the whole biological focal region that actually plays a main role in coagulative necrosis of the subject during treatment. Usually, the diameter of focal core 23 is not bigger than the section diameter (2mm-5mm) of two-dimensional image formed by the imaging device. When the subject within this region is imaged during treatment, only a two-dimensional imaging for the focal core is needed. If two-dimension images of the tumor to be treated are established and the obtained tumor contour on the image is real and clear, these images can be used for treatment and the accuracy of treatment can be ensured. Meanwhile, because the diameter of biological focal region is far larger than the diameter of focal core, if the diseased tissue is treated only according to the range of biological focal region, the coagulative necrosis of the diseased tissue is incomplete for some surgeries which need accurate treatment and it is difficult to achieve good therapeutic effects. But this defect can be eliminated by imaging the focal core.

Because said ultrasound therapeutic applicator, under the control of a motion positioning system, utilizes the focal core to scan and treat the diseased tissue, which is imaged by imaging device in two-dimensional image and also the diameter of the two-dimensional section formed by imaging device ranges from 2mm to 5mm, the diseased tissue can be imaged accurately by use of two-dimensional section if only the diameter of focal core is within the range of 2mm to 5mm.

However, the focused ultrasound therapeutic apparatuses in the prior art mainly use a B-mode scanner as an imaging device to locate the applicator and to monitor the therapy. Such B-mode ultrasound imaging device has the following disadvantages on monitoring and treating: 1. because the B-mode ultrasound image is only a plane image with a certain angle, and even though a three-dimensional ultrasound system is used, the visible area is still limited; 2. the ultrasound image is limited on the observation depth, and the bone substances influence the image greatly and the tissue behind the bone can hardly be observed and there are artifacts; 3. the ultrasound images have poor capacity in identifying the tissue boundary and particularly it is more difficult to identify small tumors and deep-bedded tumors. Therefore, such ultrasound imaging device is not suitable for all kinds of fine surgeries, however, some non-ultrasound imaging devices with a function of two-dimensional imaging have no problems stated above.

Therefore, said non-ultrasound imaging device which images the diseased tissue is a CT or an MRI device, which has the function of two-dimensional imaging. For an MRI device, because it may apply appropriate gradients to the magnetic field so that the magnetic resonance signals can be selectively acquired. The information is processed to gain the tissue characteristics of each point and the tissue can be imaged. The obtained Magnetic Resonance Image has a great ability to identify different tissues and can easily distinguish the normal tissue from the tumor tissue, and the obtained three-dimensional data within certain volume may image a part of a human body or a full body, therefore, the MRI device is very suitable to monitor the high-intensity focused ultrasound therapy.

In order to keep the structure of non-ultrasound imaging device unchanged and to make the imaging device be compatible well with the ultrasound therapeutic apparatus, said ultrasound therapeutic applicator is preferably located under the diseased tissue of a patient for treatment, therefore, there is an opening on said therapeutic bed and the ultrasound therapeutic applicator is located under the opening. The ultrasound therapeutic applicator further comprises a container, which is connected under said opening and full of degassed water, and the ultrasound transducer is located in said container.

In order to keep the level of degassed water in the container stable and to reduce the impact of level changes on the imaging results, preferably, in the present invention, the opening on the therapeutic bed is covered by a membrane with pores, which can permeate liquid. Because the membrane can limit the maximum water level, the degassed water in the container will not shake greatly and the water level change is small when the ultrasound therapeutic applicator moves. This membrane and said container constitute a half-open structure.

Said container may adopt a full open structure.

When an MRI device is used as a non-ultrasound imaging device, a first surface coil for receiving the imaging information from the imaging device is installed at one side of the patient held on the therapeutic bed. The first surface coil is fixed outside around the opening on the therapeutic bed and is connected to the imaging information receiving unit of the MRI device so that the images of the focal core in the subject can be monitored accurately.

In addition, because the diameter of the focal core is small, accurate images are required to monitor the treatment process of focal core. So, further in the present invention, a second surface coil for further receiving the imaging information from the imaging device is arranged at the other side of the patient and opposite to the first surface coil. The second surface coil is also connected to the imaging information receiving unit of the MRI device.

Guided by an imaging device, the high intensity focused ultrasound therapeutic system of the present invention utilizes the focal core to scan and treat the diseased tissue; only an imaging device having a function of two-dimensional imaging is needed for monitoring, so the imaging device needs no modification. Meanwhile, because the energy focused at the diseased tissue is relatively high when the focal core is used for treatment, and it is helpful to protect the tissue along the acoustic pathway, this function makes the system have the characteristics of a "knife" and the system of the present invention can be applied to all kinds of surgeries which need accurate treatment. In the present invention, a high intensity focused ultrasound therapeutic system guided by an imaging device which is formed by a combination of the MRI device with two-dimensional imaging function and an ultrasound transducer with the diameter of focal core ranging from 0.1mm to 5mm in the biological focal region, can reduce the production cost of this system and greatly improve the use effect and the therapeutic effects.

### BRIEF DESCRIPTION OF THE FIGURES

Fig. 1 is a principle diagram for the high intensity focused ultrasound therapy in the prior art.
Fig. 2 is a longitudinal section diagram of biological focal region.
Fig. 3 is a structural diagram of the embodiment 1 of the present invention.
Fig. 4 is a selective enlargement top view of the therapeutic bed 11 in Fig. 3.
Fig. 5 is a structural diagram of the embodiment 2 of the present invention.

Wherein: 1-Ultrasound transducer 2-Biological focal region 21-Foacal edge 22-Focal segment 23-Focal core 3-diseased tissue 4-MRI table 5-Imaging information receiving unit 6-Information processing unit 7-Display 8-Ultrasound transducer 9-Container 10-Opening 11-Therapeutic bed 12-Motion positioning system 13-Therapeutic bed controlling unit 14-First surface coil 15-MRI bore 16-Second surface coil 17-Patient

### DETAILED DESCRIPTION OF PREFERRED EMBODIMENTS

The present invention is further explained below in detail with reference to the preferred embodiments and accompanying drawings.

### Embodiment 1:

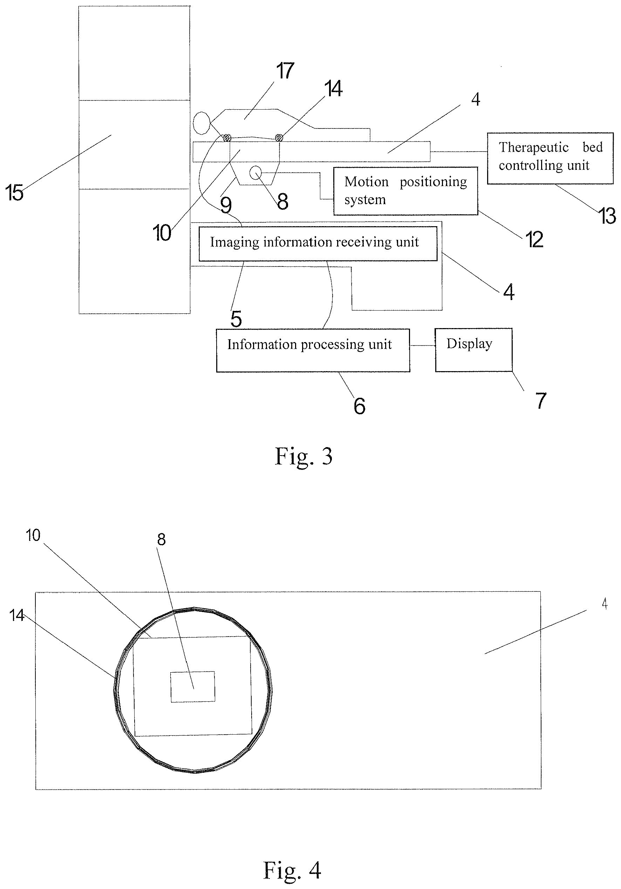

As shown in Fig. 3, the high intensity focused ultrasound therapeutic system guided by an imaging device in the present invention consists of a non-ultrasound imaging device and a high intensity focused ultrasound therapeutic device. In this embodiment, an MRI device is applied as non-ultrasound imaging device.

In the MRI device shown in Fig. 3, MRI imaging information receiving unit 5 is equipped into the MRI table 4. The imaging information receiving unit 5 is connected to information processing unit 6 and display 7 respectively.

The high intensity focused ultrasound therapeutic device comprises an ultrasound therapeutic applicator, a motion positioning system 12 of ultrasound therapeutic applicator, a therapeutic bed 11 for holding a patient and with an opening 10, and a therapeutic bed controlling unit 13 for controlling therapeutic bed 11 to move in and out of MRI bore 15.

Wherein, the ultrasound therapeutic applicator comprises an ultrasound transducer 8 and a container 9. The container 9 is connected under the opening 10 of therapeutic bed 11. In this embodiment, the container 9 is a water reservoir, which is placed at the opening 10 on therapeutic bed 11 and is full of degassed water. The degassed water is used as a medium for ultrasound transmission and also used to cool the heat produced during treatment. The ultrasound transducer 8 is located in the container 9.

The container 9 in this embodiment is an open reservoir. In order to reduce the influence on the imaging of the subject due to the level changes of the degassed water, the opening 10 on the therapeutic bed 11 can be covered by a membrane (not shown in figures) with pores, through which the liquid permeate. This membrane and said container 9 constitute a half-open structure.

In this embodiment, the ultrasound transducer 8 adopts a spherical focusing piezoelectric transducer with a focal length ranging from 80mm to 200mm, a diameter ranging from 80mm to 300mm, a diameter of focal core of biological focal region ranging from 0.2mm to 2mm and working frequency ranging from 0.5MHz to 2MHz. Certainly, in this embodiment, the phased transducer can be used as an ultrasound source after appropriate adjustment.

During treatment, the focal core 23 is used to scan and treat the target tissue, and the MRI device monitors the treatment process of the focal core by using the function of two-dimensional imaging, and the two-dimensional images are shown to an operator through a display 7.

In order to ensure the safety of two-dimensional scanning therapy of focal core 23 and to improve the definition of the image information collected by the MRI device, as shown in Fig. 4, the first surface coil 14 for receiving the imaging information from MRI device is installed at one side of the patient 17 held on the therapeutic bed 11. The first surface coil 14 is fixed outside around the opening 10 on the therapeutic bed 11 with glue or in other practicable ways and is connected to the imaging information receiving unit 5 of the MRI device. The shape of this coil is round. Because the position of the first surface coil 14 for receiving the imaging information from MRI is closer to the position of a patient to be treated, the signals received are relatively strong and the imaging quality is better and the two-dimensional images of the target scanned by focal core 23 can be monitored more accurately during treatment.

In this embodiment, besides adding the first surface coil 14, the MRI device is not modified. The imaging information receiving unit 5, information processing unit 6 and display 7 are existing components of MRI device.

The working process of high intensity focused ultrasound therapeutic system in the present embodiment is shown as follows: at first, under the control of therapeutic bed controlling unit 13, the patient 17 on the therapeutic bed 11 is moved into the MRI bore 15 and the diseased tissue of the patient 17 is located within the magnetic resonance imaging area. The first surface coil 14 is used to receive imaging information from MRI and the information received here is transmitted to the imaging information receiving unit 5 on the MRI table 4. Then the imaging information receiving unit 5 transmits the information to the information processing unit 6. After the information is processed by information processing unit 6, the two-dimensional images established for the diseased tissue to be treated are shown to an operator through the display 7. Then, under the control of motion positioning system 12, the focal core 23 of the ultrasound transducer 8 is overlapped with the diseased tissue to be treated within the magnetic resonance volume area. Driven by the driving circuit (not shown in figures), the ultrasound transducer 8 transmits ultrasonic waves and the focal core 23 is used to scan and treat the diseased tissue in the target area. Because the diameter of focal core (0.2mm-2mm) is smaller than the section diameter (2mm-5mm) of single slice image formed by MRI, only two-dimensional images of the target need to be established during treatment when MRI device monitors the treatment process. And, the established two-dimensional images are shown to an operator through a display. In the present embodiment, the focal core 23 is used to treat the diseased tissue and the temperature rise at the focal core 23 is the highest in the entire biological focal region, and the energy degression at the outer edge of the focal core 23 is very sharp and characterizes as a "knife". Therefore, the high intensity focused ultrasound therapeutic system of the present embodiment can be applied to all kinds of complicated surgeries.

It needs to be explained that the MRI guided high intensity focused ultrasound technique in this embodiment is not limited in the application scope of present invention. Personnel skilled in this art can make appropriate adjustment and modification to the technical solutions of the present invention to make it be applicable to other non-ultrasound imaging devices, for example, a CT device. The technical solutions after all such adjustment and modifications are still within the protection scope of the present invention.

### Embodiment 2:

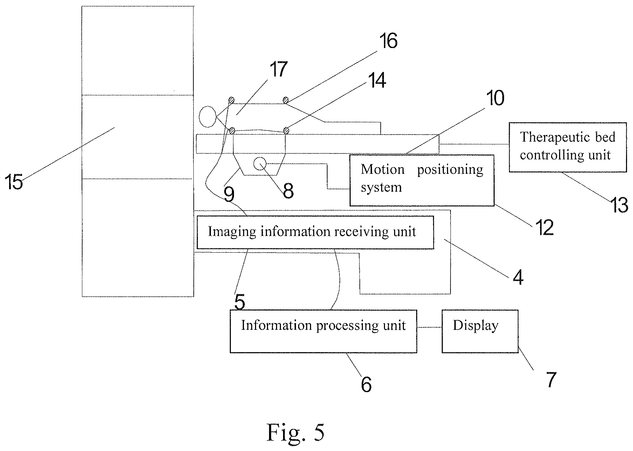

As shown in Fig. 5, the difference between this embodiment and the embodiment 1 is that the second surface coil 16 is added at the other side of the patient and opposite to the first surface coil. The second surface coil 16 and the first surface coil 14 are integrated and then connected to the imaging information receiving unit 5 of the MRI device. The purpose of adding the second surface coil 16 is to enhance the intensity to receive MRI imaging signals. Compared with the embodiment 1, this embodiment has a higher imaging quality and it is more helpful for an operator to monitor two-dimensional images of the target scanned by the focal core 23 during treatment.

The rest of structures and procedures of application are as the same in the embodiment 1, there is no need of repeated description.

## Claims

1. A high intensity focused ultrasound therapeutic system guided by an imaging device for treating diseased tissue comprising:
an ultrasound therapeutic applicator, which contains an ultrasound transducer (8), a positioning system (12) for the ultrasound therapeutic applicator, a therapeutic bed (11) for laying a patient thereon, and an imaging device (4) used for imaging the diseased tissue; wherein
said imaging device is a non-ultrasound imaging device with two-dimensional imaging function to image an image area, the ultrasound transducer having a biological focal region with focal core diameter ranging from 0.1mm to 5mm, the biological focal region comprises three parts which are focal edge, focal segment and focal core, a focal region center of the biological focal region is the focal core whose temperature rise in use is the highest, an outer edge of the focal core has sharp energy degression and having the characteristics of a "knife" **characterised in that**
the diameter of the focal core is not bigger than a section thickness of a two-dimensional image formed by the imaging device to accurately image the diseased tissue, wherein the section thickness is 2mm- 5mm, under the control of the positioning system the focal core of the ultrasound transducer is overlapped with the diseased tissue to be treated and scanned within the imaging area.

2. The ultrasound therapeutic system as claimed in claim 1, wherein said ultrasound transducer (8) with the diameter of focal core ranging from 0.2 to 2mm in the biological focal region is adopted.

3. The ultrasound therapeutic system as claimed in claim 1, wherein there is an opening (10) on said therapeutic bed (11), and the ultrasound therapeutic applicator is located under the opening.

4. The ultrasound therapeutic system as claimed in claim 3, wherein said ultrasound therapeutic applicator further comprises a container (9), which is connected under said opening (10) and full of degassed water, and the ultrasound transducer (8) is located in said container.

5. The ultrasound therapeutic system as claimed in claim 4, wherein said container (9) has a full open structure.

6. The ultrasound therapeutic system as claimed in claim 4, wherein said opening (10) on the therapeutic bed (11) is covered by a membrane with pores which covers the upper part of the container (9), forming a half-open structure.

7. The ultrasound therapeutic system as claimed in any one of claims 1-6, wherein said non-ultrasound imaging device for imaging the diseased tissue is a CT or an MRI device with the function of two-dimensional imaging.

8. The ultrasound therapeutic system as claimed in any one of claims 3-6, wherein when an MRI device is adopted for the non-ultrasound imaging device, the first surface coil (14) for receiving the imaging information from imaging device is installed at one side of the therapeutic bed for holding the patient; the first surface coil is fixed outside around the opening 10 on the therapeutic bed (11) and is connected to an imaging information receiving unit (5) of the MRI device.

9. The ultrasound therapeutic system as claimed in claim 8, wherein a second surface coil (16) for further receiving the imaging information from imaging device is arranged at the other side of the patient (17) and opposite to the first surface coil (14), and the second surface coil is connected to the imaging information receiving unit (5) of the MRI device.

## Patentansprüche

1. Hochintensität fokussiertes Ultraschall-Therapiesystem, das von einer Abbildungsvorrichtung zur Behandlung von erkranktem Gewebe geführt wird, umfassend:
einen Ultraschall-Therapieapplikator, der einen Ultraschallwandler (8) enthält, ein Positionierungssystem (12) für den Ultraschall-Therapieapplikator, ein therapeutisches Bett (11) zum Auflegen eines Patienten darauf und eine Abbildungsvorrichtung (4), die zur Abbildung des erkrankten Gewebes verwendet wird; wobei
die Abbildungsvorrichtung eine nicht-ultraschallische Abbildungsvorrichtung mit einer zweidimensionalen Abbildungsfunktion zur Abbildung eines Bildbereichs ist, wobei der Ultraschallwandler einen biologischen Fokusbereich mit einem Fokuskerndurchmesser im Bereich von 0,1 mm 5 mm aufweist, wobei der biologische Fokusbereich drei Teile umfasst, die eine Fokuskante, ein Fokussegment und ein Fokuskern sind, wobei ein Fokusbereichszentrum des biologischen Fokusbereichs ist der Fokuskern ist, dessen Temperaturanstieg im Gebrauch am höchsten ist, wobei eine Außenkante des Fokuskerns eine starke Energiedegression aufweist und die Eigenschaften eines "Messers" hat, **dadurch gekennzeichnet, dass**
der Durchmesser des Fokuskerns nicht größer ist als eine Sektionsdicke eines zweidimensionalen Bildes, das von der Abbildungsvorrichtung erzeugt wird, um das erkrankte Gewebe genau abzubilden, wobei die Sektionsdicke 2 mm bis 5 mm beträgt, wobei unter der Steuerung des Positionierungssystems der Fokuskern des Ultraschallwandlers mit dem zu behandelnden erkrankten Gewebe überlappt und innerhalb des Abbildungsbereichs gescannt wird.

2. Ultraschall-Therapiesystem nach Anspruch 1, wobei der Ultraschallwandler (8) mit dem Durchmesser des Fokuskerns im Bereich von 0,2 mm bis 2 mm im biologischen Fokusbereich angenommen wird.

3. Ultraschall-Therapiesystem nach Anspruch 1, wobei eine Öffnung (10) auf dem therapeutischen Bett (11) vorhanden ist und der Ultraschall-Therapieapplikator unter der Öffnung angeordnet ist.

4. Ultraschall-Therapiesystem nach Anspruch 3, wobei der Ultraschall-Therapieapplikator ferner einen Behälter (9) umfasst, der unter der Öffnung (10) angeschlossen und mit entgastem Wasser gefüllt ist, und der Ultraschallwandler (8) in dem Behälter angeordnet ist.

5. Ultraschall-Therapiesystem nach Anspruch 4, wobei der Behälter (9) eine vollständig offene Struktur aufweist.

6. Ultraschall-Therapiesystem nach Anspruch 4, wobei die Öffnung (10) auf dem therapeutischen Bett (11) von einer Membran mit Poren bedeckt ist, die den oberen Teil des Behälters (9) bedeckt und eine halb offene Struktur bildet.

7. Ultraschall-Therapiesystem nach einem der Ansprüche 1 bis 6, wobei die Nicht-Ultraschall-Bildgebungsvorrichtung zur Darstellung des erkrankten Gewebes ein CT oder eine MRT-Vorrichtung mit der Funktion der zweidimensionalen Bildgebung ist.

8. Ultraschall-Therapiesystem nach einem der Ansprüche 3 bis 6, wobei, wenn eine MRT-Vorrichtung für die Nicht-Ultraschall-Bildgebungsvorrichtung eingesetzt wird, die erste Oberflächenspule (14) zum Empfangen der Bildinformationen von der Bildgebungsvorrichtung auf einer Seite des therapeutischen Betts zum Halten des Patienten installiert ist; wobei die erste Oberflächenspule außerhalb der Öffnung (10) auf dem therapeutischen Bett (11) befestigt ist und mit einer Bildinformationsaufnahmeeinheit (5) der MRT-Vorrichtung verbunden ist.

9. Ultraschall-Therapiesystem nach Anspruch 8, wobei eine zweite Oberflächenspule (16) zum weiteren Empfangen der Abbildungsinformationen von der Abbildungsvorrichtung auf der anderen Seite des Patienten (17) und gegenüber der ersten Oberflächenspule (14) angeordnet ist und die zweite Oberflächenspule mit der Abbildungsinformationsaufnahmeeinheit (5) der MRT-Vorrichtung verbunden ist.

## Revendications

1. Système thérapeutique à ultrasons focalisés de haute intensité, ledit système étant guidé par un dispositif d'imagerie pour traiter un tissu malade, ledit système comprenant :
un applicateur thérapeutique à ultrasons, lequel applicateur comprend un transducteur à ultrasons (8),
un système de positionnement (12) pour l'applicateur thérapeutique à ultrasons,
un lit thérapeutique (11) pour y coucher dessus un patient, et
un dispositif d'imagerie (4) utilisé pour imager le tissu malade ;
système dans lequel ledit dispositif d'imagerie est un dispositif d'imagerie n'étant pas à ultrasons, ledit dispositif ayant une fonction d'imagerie en deux dimensions pour imager une zone d'image, le transducteur à ultrasons ayant une région focale biologique dont le diamètre de noyau focal varie suivant une plage comprise entre 0,1 mm et 5 mm, la région focale biologique comprend trois parties qui sont le bord focal, le segment focal et le noyau focal, un centre de la région focale biologique est le noyau focal dont l'augmentation de température, lors de l'utilisation, est la plus élevée, un bord extérieur du noyau focal a une forte diminution d'énergie et se présente en ayant les caractéristiques d'un "couteau",
**caractérisé**
**en ce que** le diamètre du noyau focal n'est pas plus important qu'une épaisseur de section d'une image en deux dimensions, ladite image étant formée par le dispositif d'imagerie pour imager de façon précise le tissu malade, où l'épaisseur de section est comprise entre 2 mm et 5 mm ; sous le contrôle du système de positionnement, le noyau focal du transducteur à ultrasons est recouvert par le tissu malade devant être traité et exploré dans les limites de la zone d'imagerie.

2. Système thérapeutique à ultrasons selon la revendication 1, dans lequel est adopté ledit transducteur à ultrasons (8) ayant le diamètre de noyau focal variant dans une plage comprise entre 0,2 mm et 2 mm dans la région focale biologique.

3. Système thérapeutique à ultrasons selon la revendication 1, dans lequel une ouverture (10) est placée sur ledit lit thérapeutique (11), et l'applicateur thérapeutique à ultrasons est placé au-dessous de l'ouverture.

4. Système thérapeutique à ultrasons selon la revendication 3, dans lequel ledit applicateur thérapeutique à ultrasons comprend en outre un récipient (9) qui est connecté au-dessous de ladite ouverture (10) et rempli d'eau dégazée, et le transducteur à ultrasons (8) est placé dans ledit récipient.

5. Système thérapeutique à ultrasons selon la revendication 4, dans lequel ledit récipient (9) a une structure complètement ouverte.

6. Système thérapeutique à ultrasons selon la revendication 4, dans lequel ladite ouverture (10) placée sur le lit thérapeutique (11) est recouverte par une membrane ayant des pores, laquelle membrane, qui recouvre la partie supérieure du récipient (9), forme une structure à moitié ouverte.

7. Système thérapeutique à ultrasons selon l'une quelconque des revendications 1 à 6, dans lequel ledit dispositif d'imagerie n'étant pas à ultrasons et servant à imager le tissu malade est un dispositif de tomodensitométrie - CT - ou bien un dispositif d'IRM ayant la fonction d'imagerie en deux dimensions.

8. Système thérapeutique à ultrasons selon l'une quelconque des revendications 3 à 6, dans lequel, quand un dispositif d'IRM est adopté pour le dispositif d'imagerie n'étant pas à ultrasons, la première bobine de surface (14) prévue pour recevoir l'information d'imagerie provenant du dispositif d'imagerie est installée sur un côté du lit thérapeutique pour maintenir le patient ; la première bobine de surface est fixée à l'extérieur, autour de l'ouverture (10) placée sur le lit thérapeutique (11), et est connectée à une unité de réception d'information d'imagerie (5) du dispositif d'IRM.

9. Système thérapeutique à ultrasons selon la revendication 8, dans lequel une deuxième bobine de surface (16) prévue pour recevoir elle aussi l'information d'imagerie provenant du dispositif d'imagerie est agencée de l'autre côté du patient (17) et placée à l'opposé de la première bobine de surface (14), et la deuxième bobine de surface est connectée à l'unité de réception d'information d'imagerie (5) du dispositif d'IRM.
